# EUROPÄISCHE PATENTANMELDUNG

(11) **EP 1 733 680 A1**
(43) Veröffentlichungstag der Anmeldung: **20.12.2006**
(21) Anmeldenummer: 06011292.7
(22) Anmeldetag: 31.05.2006
(51) Int. Cl.: A61B 5/0215, A61M 25/00

(54) **Druckmessleitung für invasive Blutdruckmessung**

(30) Priorität: 13.06.2005 DE 202005009293 U
(71) Anmelder: Smiths Medical Deutschland GmbH, 85614 Kirchseeon (DE)
(72) Erfinder: Weber, Jörg, 83533 Edling (DE); Beck, Bernd, 72414 Rangendingen (DE)
(74) Vertreter: von Bülow, Tam

(57) **Zusammenfassung**

Die Erfindung bezieht sich auf eine Druckmeßleitung, insbesondere für invasive Blutdruckmessung, die eine Wand aus flexiblem Material aufweist, welche ein Lumen umgibt. Die Wand besteht insbesondere aus mindestens einem inneren Schlauch (2) und einem diesen umhüllenden äußeren Schlauch (3), wobei das Material des inneren Schlauches (2) härter ist als das Material des äußeren Schlauches (3) und die Wanddicke des inneren Schlauches (2) kleiner ist als die Wanddicke des äußeren Schlauches (3). Ferner kontaktiert die Innenseite (6) des äußeren Schlauches (3) die Außenseite (5) des inneren Schlauches (2) vollflächig.

## Beschreibung

Die Erfindung bezieht sich auf eine Druckmeßleitung, insbesondere für invasive Blutdruckmessung gemäß dem Oberbegriff des Patentanspruches 1.

Bei der invasiven Blutdruckmessung wird ein Druckmeßwandler über eine schlauchartige Druckmeßleitung an eine Arterie oder eine Vene eines Patienten angeschlossen (vgl. DE 44 00 941 C1). Der Druckmeßwandler erzeugt ein elektrisches Ausgangssignal, das dem zu messenden Blutdruck entspricht. Dieses Signal wird üblicherweise auf einem Monitor angezeigt. Es wird dabei ein flüssigkeitsgefülltes Übertragungssystem geschaffen, das vom Blutdruck des Patienten periodisch angestoßen wird. Dieses Übertragungssystem leitet den Druckverlauf des Blutdruckes an den Druckmeßwandler weiter, wobei der zeitliche Verlauf des Druckes dem Arzt wichtige Informationen gibt.

Problematisch ist, inwieweit das endgültige, auf dem Monitor abgebildete Signal tatsächlich dem Verlauf des Blutdruckes entspricht. Eine maßgebliche Fehlerquelle kann dabei die Druckmeßleitung sein, die abhängig von ihrer Eigenfrequenz und Dämpfung zu einer mehr oder weniger starken Verfälschung des Druckverlaufes führt.

Bisher werden für die Druckmeßleitung flexible Schläuche verwendet, die zwar den Vorteil der einfachen Handhabung und Verlegung haben, die jedoch aufgrund ihrer Flexibilität zu einer Reduzierung der Resonanzfrequenz und damit zu einem größeren Übertragungsfehler führen.

Aufgabe der Erfindung ist es, eine Druckmeßleitung zu schaffen, die bei einfacher Handhabung keine oder nur geringe Verfälschung des Meßergebnisses bewirkt.

Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Das Grundprinzip der Erfindung liegt darin, daß die Druckmeßleitung aus mindestens einem inneren und einem diesen umhüllenden äußeren Schlauch besteht, wobei das Material des inneren Schlauches härter ist als das des äußeren Schlauches und die Dicke des inneren Schlauches kleiner ist als die des äußeren Schlauches.

Durch den härteren inneren Schlauch, in dessen Lumen sich die Flüssigkeit befindet, werden Druckimpulse nicht oder nur geringfügig verfälscht. Durch die reduzierte Elastizität des inneren Schlauches liegt die Resonanzfrequenz des Übertragungssystems relativ hoch, so daß für die Auswertung relevante Frequenzanteile des Druckverlaufes unterhalb der Resonanzfrequenz liegen, so daß das System den Druckverlauf originalgetreuer überträgt. Gleichzeitig wird aber auch eine Dämpfung angestrebt und erreicht, da Amplituden über Erhöhungen im Bereich von Resonanzfrequenzen gedämpft werden. Durch den äußeren, weicheren Schlauch bleibt gleichwohl die Flexibilität der Druckmeßleitung erhalten, so daß diese einfach verlegt und gehandhabt werden kann.

Damit ist auch die erforderliche Knickfestigkeit gewährleistet. Die beiden Schläuche sind fest miteinander vollflächig verbunden. Dabei berührt die Innenseite des äußeren Schlauches die Außenseite des inneren Schlauches vollflächig. Vorzugsweise sind beide Schläuche koextrudiert, so daß die Materialien der beiden Schläuche in einem Übergangsbereich miteinander vermischt sind.

Nach einer Weiterbildung der Erfindung kann der innere, härtere Schlauch noch eine innere Lage aus weicherem Kunststoff im Vergleich zu dem Material des inneren Schlauches haben, der eine gezielte Dämpfung verursacht. Diese innere Lage kann die Form eines weiteren Schlauches haben, der ebenfalls vollflächig mit dem inneren Schlauch in Kontakt steht und beispielsweise ebenfalls koextrudiert ist, so daß sich sein Material mit dem des inneren Schlauches in einem Übergangsbereich vermischt.

Diese innere Schicht ist je nach gewünschtem Dämpfungsverhalten dünner oder dicker als die des inneren Schlauches.

Die Länge der Druckmeßleitung kann beliebig sein, wobei selbstverständlich darauf zu achten ist, daß Resonanzfrequenz und Dämpfungsfaktor von diversen Parametern abhängen, von denen einer auch die Länge der Druckmeßleitung ist. Weitere Parameter sind der Durchmesser des Lumens, die Materialeigenschaften, insbesondere Elastizitätsmodul und Härte der Schläuche, Wandstärken der Schläuche und Verlegungsform der Druckmeßleitung, wobei diese möglichst geradlinig und ohne scharfe Krümmungsradien verlegt werden soll, um Reflektionen von Druckwellen zu vermeiden.

Bei der Wahl der Parameter und Materialien ist darauf zu achten, daß die Resonanzfrequenz des Systems möglichst hoch liegt, da diese sonst zu wesentlichen Meßfehlern oder Fehlinterpretationen des Signals führen kann, was wieder zu einer Fehlbehandlung eines Patienten führen kann. Andererseits ist aus Gründen der Anwenderfreundlichkeit und der flexiblen Verlegung der Druckmeßleitung auch darauf zu achten, daß eine ausreichende Flexibilität gewährleistet ist.

Im folgenden wird die Erfindung anhand eines Ausführungsbeispieles im Zusammenhang mit der Zeichnung ausführlicher erläutert. Es zeigt:

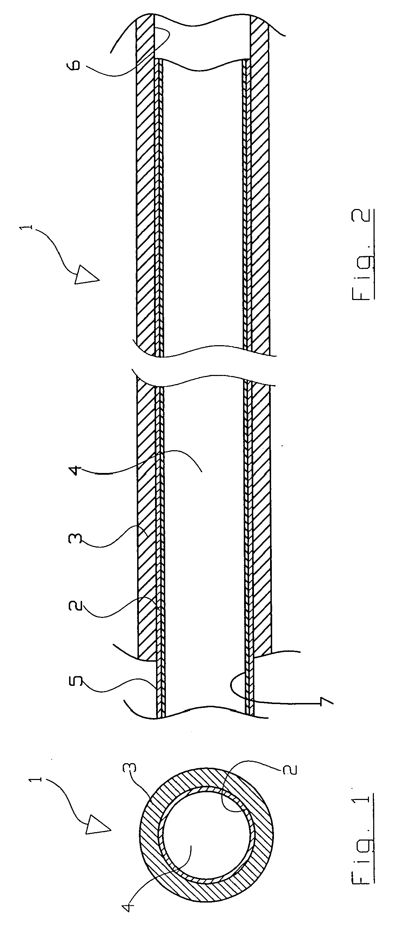

Fig. 1 einen Querschnitt der Druckmeßleitung; und
Fig. 2 einen Abschnitt der Druckmeßleitung im Längsschnitt.

Die Druckmeßleitung ist in ihrer Gesamtheit mit dem Bezugszeichen 1 bezeichnet. Sie hat mindestens einen inneren Schlauch 2 und einen diesen umhüllenden äußeren Schlauch 3. Der innere Schlauch 2 bildet ein Lumen 4, das bei der Blutdruckmessung durch einen nicht dargestellten Katheter mit einem Blutgefäß, wie z.B. einer Arterie oder einer Vene eines Patienten, verbunden ist und das mit seinem anderen Ende mit einem Druckmeßwandler verbunden ist.

Die beiden Schläuche 2 und 3 kontaktieren sich vollflächig, so daß die Außenseite 5 des inneren Schlauches 2 vollflächig die Innenseite 6 des äußeren Schlauches 3 kontaktiert. Der innere Schlauch 2 und der äußere Schlauch 3 sind aus unterschiedlichen Materialien, wobei der innere Schlauch 2 aus härterem Material ist als der äußere Schlauch 3, so daß die Schwingungsdämpfung für Druckschwingungen im Lumen 4 weitestgehend eliminiert ist. Die Materialien des inneren Schlauches 2 und des äußeren Schlauches 3 können sich in einem Übergangsbereich vermischen, was in der Praxis durch Koextrudieren beider Schläuche erreicht wird.

Zur Einstellung einer vordefinierten Dämpfung kann der innere Schlauch 2 an seiner Innenwand 7 noch eine Beschichtung 8 aus weicherem Material haben im Vergleich zum Material des inneren Schlauches 5. Auch diese Schicht 7 kontaktiert den inneren Schlauch vollflächig und kann ebenfalls durch Koextrusion erzeugt werden, so daß sich das Material der Beschichtung 7 mit dem des inneren Schlauches 2 in einem Übergangsbereich vermischt.

Die Wandstärke des inneren Schlauches 2 ist kleiner als die Wandstärke des äußeren Schlauches 3. Die Wandstärke der optionalen Beschichtung 7 ist kleiner als die des äußeren Schlauches 3 und je nach Dämpfungsverhalten kleiner, gleich oder größer als die des inneren Schlauches 2.

Bei Wahl der Materialien und der Dimensionierungen ist darauf zu achten, daß die Resonanzfrequenz möglichst hoch und die Dämpfung nicht zu niedrig ist, um im interessierenden Druck- und Frequenzbereich ein optimales Übertragungsverhalten und eine möglichst geringfügige Verfälschung des Meßergebnisses zu erreichen. Andererseits ist darauf zu achten, daß die Leitung ausreichend flexibel ist, um leicht handhabbar zu sein und frei vom Patienten zu einem Druckmeßwandler verlegt werden kann.

## Patentansprüche

1. Druckmeßleitung, insbesondere für invasive Blutdruckmessung, die eine Wand aus flexiblem Material aufweist, welche ein Lumen umgibt,
**dadurch gekennzeichnet,**
**daß** die Wand aus mindestens einem inneren Schlauch (2) und einem diesen umhüllenden äußeren Schlauch (3) besteht,
**daß** das Material des inneren Schlauches (2) härter ist als das Material des äußeren Schlauches (3),
**daß** die Wanddicke des inneren Schlauches (2) kleiner ist als die Wanddicke des äußeren Schlauches (3) und
**daß** die Innenseite (6) des äußeren Schlauches (3) die Außenseite (5) des inneren Schlauches (2) vollflächig kontaktiert.

2. Druckmeßleitung nach Anspruch 1, **dadurch gekennzeichnet,**
**daß** Materialien des inneren Schlauches (2) und des äußeren Schlauches (3) in einem Übergangsbereich miteinander vermischt sind.

3. Druckmeßleitung nach Anspruch 1 oder 2, **dadurch gekennzeichnet,**
**daß** die Innenseite des inneren Schlauches (2) mit einer Beschichtung (7) versehen ist, die weicher ist als das Material des inneren Schlauches (2).

4. Druckmeßleitung nach Anspruch 3, **dadurch gekennzeichnet,**
**daß** die Beschichtung (7) dünner ist als die Wanddicke des äußeren Schlauches (3) und vorzugsweise auch dünner als die Wandstärke des inneren Schlauches (2).
